# EUROPEAN PATENT APPLICATION

(11) **EP 3 420 904 A1**
(43) Date of publication of application: **02.01.2019**
(21) Application number: 17178936.5
(22) Date of filing: 30.06.2017
(51) Int. Cl.: A61B 6/00, A61B 90/00, A61B 34/20, G06T 7/30, A61B 5/055, A61B 34/10

(54) **DEVICE AND METHOD FOR PREDICTING AN INTRA-OPERATIONAL NON-RIGID BRAIN SHIFT DEFORMATION**

(71) Applicant: Koninklijke Philips N.V., 5656 AE Eindhoven (NL)
(72) Inventor: BUERGER, Christian, 5656 AE Eindhoven (NL); STEHLE, Thomas Heiko, 5656 AE Eindhoven (NL); HOLTHUIZEN, Ronaldus Frederik Johannes, 5656 AE Eindhoven (NL)
(74) Representative: de Haan, Poul Erik

(57) **Abstract**

The present invention relates to a device for predicting an intra-operational non-rigid brain shift deformation, the device (1) comprising: a receiving unit (10); and a modelling unit (11); wherein the receiving unit (10) is configured to receive at least one intra-operational brain image; wherein the intra-operational brain image depicts a brain having a non-rigid brain shift deformation; and wherein the modelling unit (11) is configured to apply a time-dependent model for predicting non-rigid brain shift deformations to the depicted non-rigid brain shift deformation of the intra-operational brain image. The invention captures a future development of a non-rigid brain shift deformation in open skull surgery

## Description

### FIELD OF THE INVENTION

The present invention relates to a device and a method for predicting an intra-operational non-rigid brain shift deformation.

### BACKGROUND OF THE INVENTION

In open skull brain interventions, C-arm image acquisition systems acquire intra-operational 3D computed tomography (CT) images of the head to guide the intervention. However, soft tissue contrast of brain structures in CT is poor which challenges any image processing that might be incorporated in the application, such as image segmentation or image registration.

For surgery on the left side of the brain, for example, the patient's head needs to face left side up to allow easy brain access to the surgeon. However, when opening the skull, the brain decouples from the skull and compresses non-rigidly due to the gravitational force. The poor soft tissue contrast of brain structures in CT challenges any image processing that might be needed for the intervention.

From WO 2013/116694 A1 it is known to compare pre-operational surface maps of the brain with intra-operational surface maps of the brain to determine the three-dimensional displacement of the surface features of the brain. Using those three-dimensional displacements of the surface features a computer-based model of the deformation is determined. However, that computer-based model of the deformation does not capture the future development of any non-rigid brain shift deformation.

### SUMMARY OF THE INVENTION

There may thus be a need to capture a future development of a non-rigid brain shift deformation in open skull surgery.

The object of the present invention is solved by the subject-matter of the independent claims; further embodiments are incorporated in the dependent claims. It should be noted that the following described aspects of the invention apply also for the method.

According to the present invention, a device for predicting an intra-operational non-rigid brain shift deformation comprises: a receiving unit; and a modelling unit; wherein the receiving unit is configured to receive at least one intra-operational brain image; wherein the intra-operational brain image depicts a brain having a non-rigid brain shift deformation; and wherein the modelling unit is configured to apply a time-dependent model for predicting non-rigid brain shift deformations, to the depicted non-rigid brain shift deformation of the intra-operational brain image.

The device according to the invention receives an intra-operational brain image and uses that intra-operational brain image to predict the non-rigid brain shift deformation with the time-dependent model applied by the modelling unit. The time-dependent model provides a prediction of the non-rigid brain shift deformation wherein the time-dependent model determines the non-rigid brain shift deformation by e.g. considering the gravitational force on the brain during opened skull surgery. A surgeon can therefore use the model to predict where brain structures are located after opening the skull, i.e. the position change of the brain structures during the surgery. A surgeon can therefore use that prediction to find the brain structures during the surgery. The device therefore captures a future development of a non-rigid brain shift deformation in open skull surgery.

According to an example, the device further comprises: a skull hole detector; wherein the skull hole detector is configured to detect a position, a size and a spatial orientation of a patient's skull hole in the intra-operational brain image; and wherein the modelling unit is configured to predict a future non-rigid brain shift deformation based on the intra-operational brain image with the time-dependent model based on the position, the size and the spatial orientation of a patient's skull hole.

Using the skull hole detector, the time-dependent model for predicting the non-rigid brain shift deformation may be fed with further information. That further information may be the position, the size, and/or the spatial orientation of a skull hole in a patient's skull during the surgery. The knowledge of the position, the size, and/or the spatial orientation of the skull hole may provide the time-dependent model the information where the brain in the opened skull still has contact to the inner wall of the skull and where the brain does not have any contact to an inner skull wall, i.e. at the skull hole. That further information in combination with the consideration of the gravitational force provides the time-dependent model an improved measure to predict the non-rigid brain shift deformation of the patient's brain in the open skull.

According to an example, the device further comprises: a display unit; wherein the display unit is configured to provide a modified patient's pre-operational brain image showing the predicted time-dependent non-rigid brain shift deformation.

The display unit therefore provides the surgeon with the information about the prediction of the non-rigid brain shift deformation being determined by the time-dependent model of the modelling unit.

According to an example, the time-dependent model is a soft tissue model; and the modelling unit is configured to adapt parameters of the soft tissue model when applying the time-dependent model of non-rigid brain shift deformation.

According to another example, the modelling unit is configured to perform a segmentation on the intra-operational brain image; wherein the modelling unit is configured to establish a model of the non-rigid brain shift deformation based on the segmentation when applying the time-dependent model of non-rigid brain shift deformation.

According to a further example, the intra-operational brain image is an image having a low soft tissue contrast, e.g. computed tomography brain image or a cone beam computed tomography image.

The intra-operational brain image which is used for the application of the time-dependent model therefore does not need to provide a high soft tissue contrast. For the application of the time-dependent model it is sufficient that the intra-operational brain image provides the skull structure and the location of the skull cavity. The use of an image having a high soft tissue contrast, e.g. the use of a magnetic resonance image, is not excluded.

According to another example, the time-dependent model determines the effect of the gravitational force on a brain being depicted in the intra-operational brain image.

According to another example, the time-dependent model determines model considers the leakage of cerebral fluid and/or the adherence of the brain on the inner skull bone layer in the intra-operational brain image for the prediction of the non-rigid brain shift deformation.

The consideration of the leakage of cerebral fluid and/or the adherence of the brain on the inner skull bone layer further improves the prediction quality of the non-rigid brain shift deformation.

According to an example, the device further comprises: a comparator; wherein the receiving unit is configured to receive at least one training pre-operational brain image and at least one training intra-operational brain image, the at least one training pre-operational brain image and the at least one training intra-operational brain image having a high soft tissue contrast; wherein the comparator is configured to compare the at least one training pre-operational brain image and at least one training intra-operational brain image resulting in a determined non-rigid brain shift deformation; wherein the modelling unit is configured to develop the time-dependent model for predicting a non-rigid brain shift deformation based on the determined non-rigid brain shift deformation.

Using the comparator, the modelling unit may establish the time-dependent model for predicting a non-rigid brain shift deformation. The modelling unit feeds the determined non-rigid brain shift deformation from the comparison between the pre-operational brain image and the intra-operational brain image into a time-dependent model. Due to that feeding of the information to the time-dependent model, the modelling unit may adapt the time-dependent model to the determined brain shift deformation. The adaption of the time-dependent model changes the time-dependent model such that a prediction of the brain shift deformation may be provided by the time-dependent model, i.e. an extrapolation of the brain shift deformation. This means, that the time-dependent model learns from the determined non-rigid brain shift deformations how non-rigid brain shift deformations evolve. Furthermore, the modelling unit may consider the position, the size, and/or the spatial orientation of the skull hole in the received intra-operational brain images to adapt the time-dependent model. Thus, the time-dependent model considers the gravitational force, the position, the size, and/or the spatial orientation to learn how brain shift deformations evolve. Thus, the time-dependent model may predict brain shift deformations from intra-operational brain images without knowing the corresponding pre-operational brain image of the patient. This learning effect is independent on the patient. That means that the training images and images for the application may be taken from different people

According to an example, the at least one training pre-operational brain image and the at least one training intra-operational brain image are images having a high soft tissue contrast, e.g. magnetic resonance brain images.

Magnetic resonance brain images comprise a high soft tissue contrast such that the brain structures can be determined in the training pre-operational brain image and the training intra-operational brain image. The comparator can therefore assess the differences of the brain structure before the opening of the skull and after opening of the skull.

According to the present invention, also a system for predicting an intra-operational non-rigid brain shift deformation comprises: an imaging apparatus; and a device according to the above description; wherein the imaging apparatus is configured to acquire a current patient's brain image and provide the current patient's brain image as an intra-operational brain image.

In an example, an image processor may receive the predicted time-dependent non-rigid brain shift deformation. The image processor may process current patient's intra-operational images wherein the processing is based on the predicted time-dependent non-rigid brain shift deformation.

In an example, the device comprises a processing unit which controls the receiving unit and the modelling unit. The processing unit may further control the skull hole detector and the display unit.

According to the present invention, also a method for predicting an intra-operational non-rigid brain shift deformation comprises the following steps: a) receiving at least one intra-operational brain image with a receiving unit, wherein the intra-operational brain image depicts a brain having a non-rigid brain shift deformation; and b) applying a time-dependent model for predicting non-rigid brain shift deformations with a modelling unit, the time-dependent model being applied to the depicted non-rigid brain shift deformation of the intra-operational brain image.

The method according to the invention receives an intra-operational brain image and uses that intra-operational brain image to predict the non-rigid brain shift deformation with the time-dependent model applied by the modelling unit. The time-dependent model provides a prediction of the non-rigid brain shift deformation wherein the time-dependent model determines the non-rigid brain shift deformation by considering the gravitational force on the brain in the opened skull. A surgeon can therefore use the model to see where brain structures are located after opening the skull. Furthermore, the method provides a prediction of the position change of the brain structures during the surgery. A surgeon can therefore use that prediction to find the brain structures during the surgery. The method therefore captures a future development of a non-rigid brain shift deformation in open skull surgery.

According to the present invention, also a computer program element for controlling an apparatus according to the above description, when being executed by a processing unit, is adapted to perform the method steps according to the above description.

According to the present invention, also a computer readable medium has stored the program element according to the above description.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic drawing of a device for predicting an intra-operational non-rigid brain shift deformation.
Fig. 2 shows a schematic drawing of a system for predicting an intra-operational non-rigid brain shift deformation.
Fig. 3 shows a schematic flow chart of the method for predicting an intra-operational non-rigid brain shift deformation.
Figs. 4a, b show embodiments of a step of the method.

### DETAILED DESCRIPTION OF EMBODIMENTS

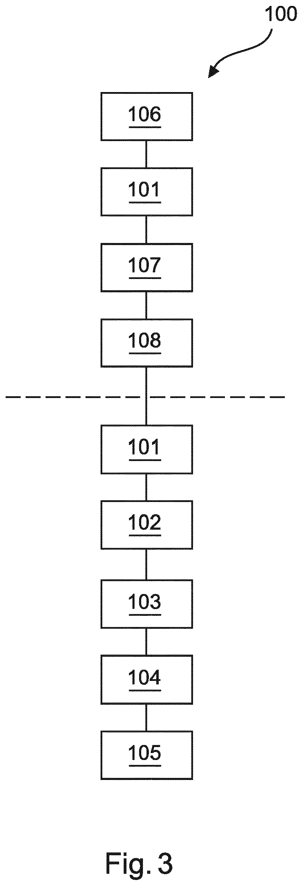

Before further describing the imaging system and the device, examples of a method are described in further detail referring to Fig. 3.

Fig. 3 shows a flowchart of the method 100. The flowchart comprises two sections being separated by the dashed line. The first section comprises steps 106, 107, and 108, wherein the second section comprises steps 101 to 105. The first section shows the training of the time-dependent model, wherein the second section shows the application of the time-dependent model.

The training of the time-dependent model for predicting non-rigid brain shift deformation starts with step 106, the receiving of at least one training pre-operational brain image with the receiving unit. The training pre-operational brain image shows the brain with the patient's skull before the surgery. This means that the patient's skull has not been opened by the surgeon, yet. Since the training pre-operational brain image shall have a high soft-tissue contrast, the training pre-operational brain image may for example be a magnetic resonance image.

Furthermore, the training may comprise step 101, i.e. receiving at least one training intra-operational brain image with a receiving unit. The training intra-operational brain image shows a brain having a non-rigid brain shift deformation. The training intra-operational brain image and the training pre-operational brain image are taken from the same patient. In step 101, the training intra-operational brain image may be received from an image acquisition system during a surgery or from a data storage, i.e. from a past surgery. The training intra-operational brain image may be an image having a good soft-tissue contrast. This means, the training intra-operational brain image may for example be a magnetic resonance image.

The training pre-operational brain image and the training intra-operational brain image are compared in step 107. The comparison determines the non-rigid brain shift deformation which happened between the acquisition of the training pre-operational brain image and the training intra-operational brain image of the patient.

In step 108 the acquired determined brain shift deformation of the patient is used to develop a time-dependent model for the simulation of the non-rigid brain shift deformation. The development is performed by a modelling unit. The developed time-dependent model considers the determined brain shift deformation between the training pre-operational brain image and the training intra-operational brain image and establishes a simulation of the brain shift deformation. Due to that establishment, the time-dependent model may predict a future brain shift deformation by analyzing an intra-operational brain image during an open skull surgery. The model can be generated, for instance, by learning the relation between skull holes and correlated typical deformations based on segmentations on pre-operational and intra-operational image pairs. In an embodiment of the method, this training is carried out on images with good soft tissue contrast such as pre-operational magnetic resonance images and intra-operational magnetic resonance images. The model can then be applied to intra-operational images in another session which do not need pre-operational images, i.e. the trained deformations can then be correlated to an automated detection of the skull hole in terms of location, size, head orientation, and time. The model further considers the increase of the brain shift over time due to the leak out of cerebral fluid is leaking out. The model therefore considers the leakage of cerebral fluid and/or the adherence of the brain on the inner skull bone layer.

The second section of the method 100, in which the time-dependent model is applied, starts with step 101 by receiving an intra-operational brain image of a patient during an open skull surgery. The second section does not need to receive a pre-operational brain image of the patient in advance to the surgery. Furthermore, based on training the non-rigid brain shift deformation may be predicted, even if the intra-operational brain image does not have a good soft-tissue contrast. Therefore, by applying the time-dependent model during a surgery, a computer tomography image which does not have a good soft-tissue contrast may be used for the prediction, i.e. as the pre-operational brain image.

In step 102, the time-dependent model for predicting non-rigid brain shift deformation is applied by the modelling unit. By providing the time-dependent model, the method is enabled to predict a non-rigid brain shift deformation during an open skull surgery.

In step 103, a skull hole is detected by a skull hole detector in the intra-operational brain image. The detection includes the position, the size, and/or the spatial orientation of the skull hole in the patient's skull. The information about the position, the size, and/or the spatial orientation of the skull hole is provided to the modelling unit, which feeds that information into the time-dependent model. The skull hole detector may detect the position, the size, and/or the orientation of the skull hole of a patient automatically.

The time-dependent model then predicts the future non-rigid brain shift deformation of a patient's brain based on that further information in step 104. Furthermore, the time-dependent model considers the gravitational force on the brain. Furthermore, the model determines a global brain shift and a local brain deformation. The global brain shift may be an anisotropic scaling of the brain. The local brain deformation may be a bump in the brain tissue. Further, the model may determine a compression of the brain in an opened skull.

In step 105, the predicted brain shift deformation is shown by a display unit in a modified patient's intra-operational brain image. The surgeon can then analyze the prediction of the time-dependent model and use that prediction for the interoperation to surgery decisions.

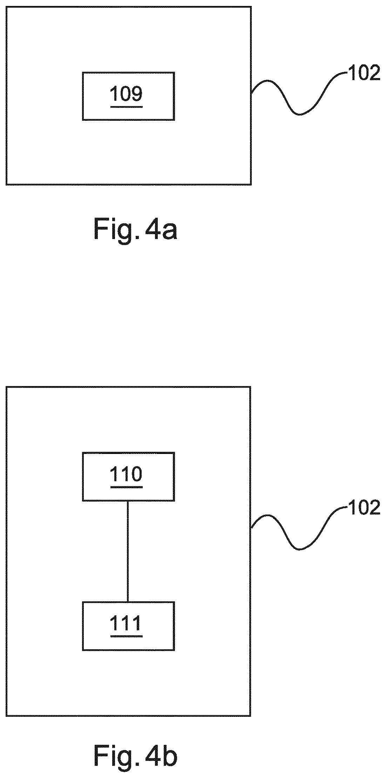

Figures 4a and 4b show substeps of the provisioning of the time-dependent model.

In a first embodiment, according to figure 4a, the time-dependent model is a soft-tissue model. The parameters of the soft-tissue model are adapted when providing the time-dependent model of non-rigid brain shift deformations in step 109.

In a second embodiment, according to figure 4b, the intra-operational brain image is segmented by the modelling unit in step 110, then the model of the non-rigid brain shift deformation is established based on the segmentation. The model is then provided as the time-dependent model of the non-rigid branch of deformation by the modelling unit.

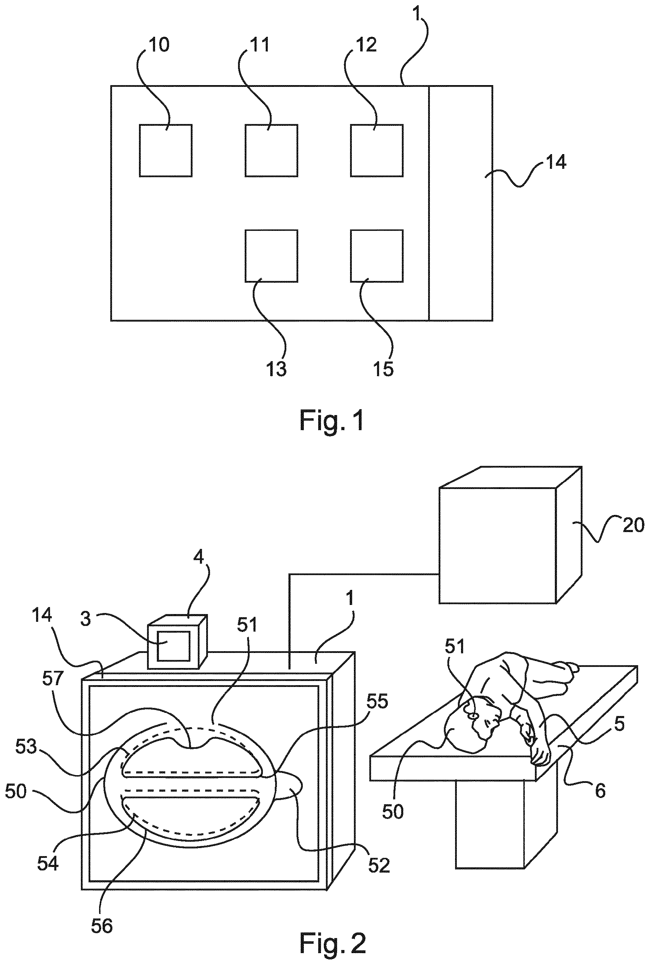

With reference to figure 1, the device 1 comprises a receiving unit 10, a modelling unit 11, a skull hole detector 12, a comparator 13, a display unit 14, and a processing unit 15.

The receiving unit receives at least one intra-operational brain image. The intra-operational image may be a training intra-operational brain image. Furthermore, the receiving unit 10 may receive a training pre-operational brain image.

The modelling unit 11 applies a time-dependent model for predicting non-rigid brain shift deformations. That model may be trained for applying the model during the surgery. The training of the time-dependent model for predicting non-rigid brain shift deformation starts with receiving of at least one training pre-operational image with the receiving unit. The training pre-operational image of the brain shows the brain with the patient's skull 50 before the surgery. This means that the patient's skull 50 has not been opened by the surgeon, yet. Since the training pre-operational brain image shall have a high soft-tissue contrast, the training pre-operational brain image may for example be a magnetic resonance image.

Furthermore, the training may comprise receiving at least one training intra-operational brain image with the receiving unit 10. The training intra-operational brain image shows a brain showing a non-rigid brain shift deformation. The training intra-operational brain image and the training pre-operational brain image are taken from the same patient 5. The training intra-operational brain image may be received from an image acquisition system during a surgery or from a data storage, i.e. from a past surgery. The training intra-operational brain image may be an image having a good soft-tissue contrast. This means, the training intra-operational brain image may for example be a magnetic resonance image.

The training pre-operational brain image and the training intra-operational brain image are compared by the comparator 13. The comparison determines the non-rigid brain shift deformation which happened between the acquisition of the training pre-operational brain image and the training intra-operational brain image of the patient.

The acquired determined brain shift deformation of the patient is used to develop a time-dependent model for the simulation of the non-rigid brain shift deformation. The development is performed by the modelling unit 11. The developed time-dependent model considers the determined brain shift deformation between the training pre-operational brain image and the training intra-operational brain image and establishes a simulation of the brain shift deformation. Due to that establishment, the time-dependent model may predict a future brain shift deformation by analyzing an intra-operational brain image during an open skull surgery. That intra-operational brain image may have a low soft tissue contrast, e.g. a cone beam computed tomography image. The model can for example be generated by learning the relation between skull holes 51 and correlated typical deformations based on segmentations on pre-operational and intra-operational image pairs.

In an embodiment of the device, this training is carried out on images with good soft tissue contrast such as pre-operational magnetic resonance images and intra-operational magnetic resonance images. The model can then be applied to intra-operational images in another session which do not need pre-operational images, i.e. the trained deformations can then be correlated to an automated detection of the skull hole in terms of location, size, head orientation, and time. The model further considers the increase of the brain shift over time due to the leak out of cerebral fluid is leaking out. The model therefore considers the leakage of cerebral fluid and/or the adherence of the brain on the inner skull bone layer.

The application of the device 1 during a surgery, in which the time-dependent model is applied, starts with receiving an intra-operational brain image of a patient 5 during an open skull surgery. During the application of the device 1, the device 1 does not need to receive a pre-operational brain image of the patient in advance to the surgery. Furthermore, based on training the non-rigid brain shift deformation may be predicted, even if the intra-operational brain image does not have a good soft-tissue contrast. Therefore, by applying the time-dependent model during a surgery, a computer tomography image which has a low soft-tissue contrast maybe used for the prediction.

The time-dependent model for predicting non-rigid brain shift deformation is applied by the modelling unit. By providing the time-dependent model, the device is enabled to predict a non-rigid brain shift deformation during an open skull surgery.

A skull hole 51 is detected by the skull hole detector 12 in the intra-operational brain image. The detection includes the position, the size, and/or the spatial orientation of the skull hole 51 in the patient's skull 50. The information about the position, the size, and/or the spatial orientation of the skull hole 51 is provided to the modelling unit 11, which feeds that information into the time-dependent model. The skull hole detector may detect the position, the size, and/or the orientation of the skull hole 51 of a patient 5 automatically.

The time-dependent model then predicts the future non-rigid brain shift deformation of a patient's brain based on that further information. Furthermore, the time-dependent model considers the gravitational force on the brain. Furthermore, the model determines a global brain shift and a local brain deformation. The global brain shift may be an anisotropic scaling of the brain. The local brain deformation may be a bump in the brain tissue. Further, the model may determine a compression of the brain in an opened skull.

The predicted brain shift deformation is shown by a display unit 14 in a modified patient's intra-operational brain image. The surgeon can then analyze the prediction of the time-dependent model and use that prediction for the interoperation to surgery decisions. This is further shown in figure 2.

In a first embodiment, the time-dependent model is a soft-tissue model. The parameters of the soft-tissue model are adapted when providing the time-dependent model of non-rigid brain shift deformations.

In a second embodiment, the intra-operational brain image is segmented by the modelling unit, then the model of the non-rigid brain shift deformation is established based on the segmentation. The model is then provided as the time-dependent model of the non-rigid branch of deformation by the modelling unit.

Figure 2 shows a system 2 comprising an imaging apparatus 20 and the device 1. The imaging apparatus 20 images a skull 50 of the patient 5. The skull 50 comprises a skull hole 51.

Figure 2 further shows that the display unit 14 shows an intra-operational image of the patient's skull 50. The intra-operational images modified with the predicted brain shift deformation. The image shows the skull hole 51 in the skull 50. The nose 52 is further shown as orientation for the surgeon.

Furthermore, the image shows the left preoperational brain hemisphere 53 and the right preoperational brain hemisphere 54. The deformed brain hemispheres 55 and 56 are overlaid in that image. The right displaced brain hemisphere 56 is displaced and deformed due to the gravitational force of the left brain hemisphere 55 which is positioned above the right brain hemisphere 56. Furthermore, the displaced left brain hemisphere 55 shows a brain deformation 57 which shows up as a bump in the left brain hemisphere 55.

In another exemplary embodiment of the present invention, a computer program or a computer program element 3 is provided, that is characterized by being adapted to execute the method steps of the method according to one of the preceding embodiments, on an appropriate system.

The computer program element 3 might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor 15 may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium for, such as a CD-ROM, is presented wherein the computer readable medium has a computer program element 3 stored on it which computer program element 3 is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device for predicting an intra-operational non-rigid brain shift deformation, the device (1) comprising:
- a receiving unit (10); and
- a modelling unit (11);
wherein the receiving unit (10) is configured to receive at least one intra-operational brain image;
wherein the intra-operational brain image depicts a brain showing a non-rigid brain shift deformation; and
wherein the modelling unit (11) is configured to apply a time-dependent model for predicting non-rigid brain shift deformations to the depicted non-rigid brain shift deformation of the intra-operational brain image.

2. Device according to claim 1, wherein the device further comprises:
- a skull hole detector (12);
wherein the skull hole detector (12) is configured to detect a position, a size and a spatial orientation of a patient's skull hole (51) in the intra-operational brain image; and
wherein the modelling unit (11) is configured to predict a future non-rigid brain shift deformation based on the intra-operational brain image with the time-dependent model based on the position, the size and the spatial orientation of a patient's skull hole (51).

3. Device according to claim 2, wherein the device further comprises:
a display unit (14);
wherein the display unit (14) is configured to provide a modified patient's pre-operational brain image showing the predicted time-dependent non-rigid brain shift deformation.

4. Device according to one of claims 1 to 3, wherein the time-dependent model is a soft tissue model; and
wherein the modelling unit (11) is configured to adapt parameters of the soft tissue model when applying the time-dependent model of non-rigid brain shift deformations.

5. Device according to one of claims 1 to 3, wherein the modelling unit (11) is configured to perform a segmentation on the intra-operational brain image;
wherein the modelling unit (11) is configured to establish a model of the non-rigid brain shift deformation based on the segmentation when applying the time-dependent model of non-rigid brain shift deformations.

6. Device according to one of claims 1 to 5, wherein the intra-operational brain image is a computed tomography brain image or a cone beam computed tomography image.

7. Device according to one of claims 1 to 6, wherein the time-dependent model determines the effect of the gravitational force on a brain being depicted in the intra-operational brain image.

8. Device according to one of claims 1 to 7, wherein the time-dependent model determines model considers the leakage of cerebral fluid and/or the adherence of the brain on the inner skull bone layer in the intra-operational brain image for the prediction of the non-rigid brain shift deformation.

9. Device according to one of claims 1 to 8, wherein the device further comprises:
- a comparator (13);
wherein the receiving unit (10) is configured to receive at least one training pre-operational brain image and at least one training intra-operational brain image, the at least one training pre-operational brain image and the at least one training intra-operational brain image providing a high soft tissue contrast;
wherein the comparator (13) is configured to compare the at least one training pre-operational brain image and the at least one training intra-operational brain image resulting in a determined non-rigid brain shift deformation;
wherein the modelling unit (11) is configured to develop the time-dependent model for predicting a non-rigid brain shift deformation based on the determined non-rigid brain shift deformation.

10. Device according to claim 9, wherein the at least one training pre-operational brain image and the at least one training intra-operational brain image are magnetic resonance brain images.

11. A system (2) for predicting an intra-operational non-rigid brain shift deformation, comprising:
- an imaging apparatus (20); and
- a device (1) according to one of the preceding claims;
wherein the imaging apparatus (20) is configured to acquire a current patient's brain image and provide the current patient's brain image as an intra-operational brain image.

12. A system according to claim 11, wherein the imaging apparatus is a computed tomography apparatus or a cone beam computed tomography apparatus.

13. A method for predicting an intra-operational non-rigid brain shift deformation, the method (100) comprising the following steps:
a) receiving (101) at least one intra-operational brain image with a receiving unit, wherein the intra-operational brain image depicts a brain having a non-rigid brain shift deformation; and
b) applying (102) a time-dependent model for predicting non-rigid brain shift deformations with a modelling unit, the time-dependent model being applied to the depicted non-rigid brain shift deformation of the intra-operational brain image.

14. A computer program element (3) for controlling an apparatus according to one of the claims 1 to 12, which, when being executed by a processing unit (15), is adapted to perform the method steps of claim 13.

15. A computer readable medium (4) having stored the program element (3) of claim 14.
